# EUROPÄISCHE PATENTSCHRIFT

(11) **EP 1 259 811 B1**
(45) Veröffentlichungstag und Bekanntmachung des Hinweises auf die Patenterteilung: **07.04.2010**
(21) Anmeldenummer: 01917034.9
(22) Anmeldetag: 26.02.2001
(51) Int. Cl.: G01N 33/569, G01N 33/50, G01N 33/68

(54) **CHEMOTAXIS-DIAGNOSTIKUM**
DIAGNOSTIC AGENT FOR CHEMOTAXIS
DIAGNOSTIC DE CHIMIOTAXIE

(30) Priorität: 28.02.2000 DE 10009420; 07.12.2000 DE 10060880
(43) Veröffentlichungstag der Anmeldung: 27.11.2002
(73) Patentinhaber: ORPEGEN Pharma Gesellschaft für biotechnologische Forschung, Entwicklung und Produktion m.b.H., 69115 Heidelberg (DE)
(72) Erfinder: NEBE, Carl, Thomas, 68526 Ladenburg (DE); HARTMANN, Karin, 67158 Ellerstadt (DE)
(74) Vertreter: Roth, Carla
(86) Internationale Anmeldenummer: PCT/EP2001/002176
(87) Internationale Veröffentlichungsnummer: WO 2001/065260

(56) Entgegenhaltungen:
- WHEELER J G ET AL: "Comparison of colony stimulation factors on in vitro rat and human neutrophil function." BIOLOGY OF THE NEONATE, (1994) 66 (4) 214-20., XP001020444
- KINSELLA I ET AL: "CHEMOTACTIC MIGRATION OF POLYMORPHONUCLEAR LEUCOCYTES PMNS ACROSS A COLLAGEN MEMBRANE" JOURNAL OF DENTAL RESEARCH, Bd. 71, Nr. SPEC. ISSUE, 1992, Seite 580 XP001023884 ISSN: 0022-0345
- TSANG, Y. T. ET AL.: "Synergy Between L-Selectin Signaling and Chemotactic Activation During Neutrophil Adhesion and Transmigration" JOURNAL OF IMMUNOLOGY, Bd. 159, Nr. 9, 1. November 1997 (1997-11-01), Seiten 4566-4577, XP001015732
- MOSER R ET AL: "Chemotaxins inhibit neutrophil adherence to and transmigration across cytokine-activated endothelium: correlation to the expression of L-selectin." EUROPEAN JOURNAL OF IMMUNOLOGY, (1993 JUL) 23 (7) 1481-7., XP001023156
- WANG, Y. ET AL.: "Functional and phenotopic characterization of monoclonal antibodies to bovine L-selectin" AMERICAN JOURNAL OF VETERINARY RESEARCH, Bd. 58, Nr. 12, Dezember 1997 (1997-12), Seiten 1392-1401, XP001020445
- YONG KWEE L: "Granulocyte colony-stimulating factor (G-CSF) increases neutrophil migration across vascular endothelium independent of an effect on adhesion: Comparison with granulocyte-macrophage colony-stimulating factor (GM-CSF)." BRITISH JOURNAL OF HAEMATOLOGY, Bd. 94, Nr. 1, 1996, Seiten 40-47, XP001023856 ISSN: 0007-1048
- RICHARDS K L ET AL: "A MODIFIED MICROCHAMBER METHOD FOR CHEMOTAXIS AND CHEMOKINESIS" IMMUNOLOGICAL COMMUNICATIONS, NEW YORK, NY, US, Bd. 13, Nr. 1, 1984, Seiten 49-62, XP002047869 ISSN: 0090-0877
- WORKU MULUMEBET ET AL: "Effect of in vitro and in vivo migration of bovine neutrophils on binding and expression of Fc receptors for IgG-2 and IgM." AMERICAN JOURNAL OF VETERINARY RESEARCH, Bd. 55, Nr. 2, Februar 1994 (1994-02), Seiten 221-226, XP001023851 ISSN: 0002-9645
- FALK W ET AL: "A 48 WELL MICRO CHEMO TAXIS ASSEMBLY FOR RAPID AND ACCURATE MEASUREMENT OF LEUKOCYTE MIGRATION" JOURNAL OF IMMUNOLOGICAL METHODS, Bd. 33, Nr. 3, 1980, Seiten 239-248, XP002048068 ISSN: 0022-1759

## Beschreibung

Die Erfindung betrifft ein Verfahren zur Bestimmung der Chemotaxis-Aktivität von Leukozyten, insbesondere Granulozyten in einer Probe sowie ein dafür geeignetes Reagenzienkit. Weiterhin betrifft die Erfindung ein Verfahren zur Diagnose eines Immundefekts oder/und einer Granulozytenfunktionsstörung.

Ein Säugerorganismus verfügt zur Abwehr von eindringenden Fremdkörpern, wie etwa Mikroorganismen, sowie zur Beseitigen von alten und beschädigten Zellen oder Zelltrümmern über Mechanismen, die zur Auflösung dieser unerwünschten Bestandteile führen. Zunächst erfolgt eine Opsonisierung der Bakterien oder Zellen, wodurch deren Phagozytose durch Leukozyten stimuliert wird. Die zur Phagozytose fähigen Leukozyten bewegen sich zunächst auf den eingedrungenen Fremdkörper zu, ein Vorgang, der als Chemotaxis bezeichnet wird. Die Chemotaxis kann durch ein Konzentrationsgefälle bestimmter Reizstoffe, wie z.B. mikrobieller Bestandteile, Komplementzerfallsprodukten oder Zytokinen, die mit spezifischen Rezeptoren, so genannten Chemorezeptoren, erkannt werden, ausgelöst werden. Chemotaktisch wirksame Stoffe bewirken zumeist eine Bewegung in Richtung der höheren Reizstoffkonzentration (positive Chemotaxis), in seltenen Fällen auch eine Bewegung von ihr weg (negative Chemotaxis). Dann erfolgt eine Adsorption, d.h. die unerwünschten Bestandteile werden an der Oberfläche der zur Phagozytose befähigten Leukozyten, im Wesentlichen Monozyten, Makrophagen und polymorphkernigen Leukozyten, gebunden. Bei der nachfolgenden Phagozytose wird der als fremd erkannte Bestandteil von der Zelle umschlossen und anschließend durch Degradation vernichtet.

J.G. Wheeler et al., Biol. Neonate 66(2) (1994) 214-220, beschreibt die Effekte von Zytokinen, z.B. G-CSF oder GM-CSF auf polymorphonukleäre Leukozyten unterschiedlicher Herkunft.

Kinsella et al., J. Dent. Research 71 (1992) 580, beschreibt ein Verfahren zur Bestimmung der chemotaktischen Bewegung von polymorphonukleären Leukozyten (PMN) mittels einer Boyden-Kammer.

Tsang et al., J. Immunol. 159(9) (1997) 4566-4577, beschreibt eine transmembrane Signalfunktion von L-Selektin in Leukozyten.

Moser et al., Eur. J. Immunol. 23 (1993) 1481-1487, beschreibt die Hemmung der Adhäsion bzw. der Transmigration von Neutrophilen durch Chemotaxine.

Wang et al., Am. J. Vet. Research 58(12) (1997) 1392-1401, beschreibt einen Chemotaxis-Assay, der in einer Boyden-Kammer durchgeführt wird.

Yong et al., Britsh Journal of Haematology, 94 (1996) 40-47, beschreibt den Einfluss von G-CSF auf die Migration bzw. Transmigration von Neutrophilen durch Endothelien sowie den Effekt von G-CSF auf die Expression von Adhäsionsmolekülen bzw. auf die Adhäsion.

Um die Leistung des Immunsystems insgesamt zu beurteilen, müssen Zahl und Funktion der Leukozyten analysiert werden. Die Zählung mit Hilfe eines differenzierenden Hämatologie automaten gegebenenfalls unter zusätzlichem Einsatz monoklonaler Antikörper ist heute bereits klinische Routine. Allerdings besteht auf Grund der zahlreichen unterschiedlichen Funktionen der Leukozyten im Rahmen der Immunabwehr noch Bedarf, die bestehenden Testmöglichkeiten durch Funktionsanalysen zu ergänzen.

Die erste Linie der Immunabwehr stellen, wie oben erläutert, die phagozytären Zellen dar und ihre Prüfung wurde im Vergleich zur Untersuchung der Lymphozyten bisher weitgehend vernachlässigt. Die in der Literatur beschriebenen Verfahren zur Analyse phagozytärer Leistung, beispielsweise die Auszählung im Mikroskop, sind im klinischen Laboralltag unpraktisch, da sie arbeitsaufwendig und in der Bewertung subjektiv sind. Bei dem so genannten Boydenkammer-Test müssen die Leukozyten einem Konzentrationsgradienten eines Chemotaxins folgend eine Membran durchwandern. Mikroskopisch ausgewertet wird dann die Entfernung der Wanderungsfront bzw. die Zellen an der Unterseite der Membran. Weiterhin ist im Stand der Technik die Messung des Formwandels von neutrophilen Granulozyten beschrieben, welcher auch zytometrisch sichtbar wird. Auf Grund der mit der Durchführung dieser Tests verbunden Schwierigkeiten war die Untersuchung auf Chemotaxis bisher jedoch Speziallabors vorbehalten.

Darüber hinaus können derzeit nur weit weniger als 50 % der klinisch beobachteten Immundefekte im Labor diagnostiziert werden.

Eine Aufgabe der vorliegenden Erfindung war es deshalb, ein Verfahren bereitzustellen, mit welchem weitere Leukozytenfunktionen, insbesondere die Chemotaxis, untersucht werden können.

Diese Aufgabe wird erfindungsgemäß gelöst durch ein Verfahren zur Bestimmung der Chemotaxis-Aktivität von Leukozyten in einer Probe, welches dadurch gekennzeichnet ist, dass man die Zahl von durch eine poröse Membran gewanderter Leukozyten durch Zugabe von Latexbeads in einem Durchflusszytometer bestimmt.

Zur Bestimmung der Chemotaxis-Aktivität von Leukozyten in einer Probe wird weiterhin bevorzugt die Expression und insbesondere die Änderung der Expressionsdichte eines Zelladhäsionsmoleküls bestimmt.

Der Test auf Chemotaxis reiht sich ein in eine Serie anderer Tests, die zur Abklärung der Leukozytenfunktion bei der Frage eines Immundefekts oder einer Immunstimulation notwendig sind. Es wurde festgestellt, dass sich der Phagozytoseprozess in verschiedene Teilschritte, wie etwa Opsonisierung, Chemotaxis, Adsorption, Phagozytose, Vernichtung und Antigenpräsentation zerlegen lässt. Diese Teilschritte können dann einzeln überprüft werden, wodurch die verschiedenen Funktionen der Leukozyten analysiert und bestimmt werden können.

Die Chemotaxis ist bei Granulozytenfunktionsstörungen relativ häufig betroffen und soll bei etwa 50 % der angeborenen Immundefekte beeinträchtigt sein.

Es wurde nun überraschenderweise festgestellt, dass parallel zur Chemotaxis-Funktion von Leukozyten und insbesondere von Granulozyten eine Änderung der Expressiondichte von bestimmten Zelladhäsionsmolekülen der Leukozyten auftritt. Durch Bestimmung oder Analyse der Expression eines Zelladhäsionsmoleküls kann damit ein für den klinischen Alltag der Laborroutine geeigneter, reproduzierbarer Test bereitgestellt werden, der eine vergleichsweise einfache Quantifizierung der Chemotaxis-Funktion von Leukozyten, insbesondere von Granulozyten, erlaubt. Das erfindungsgemäße Verfahren ist mit Hilfe der Durchflusszytometrie objektiv quantifizierbar und reproduzierbar.

Mit dem erfindungsgemäßen Verfahren kann die Chemotaxis-Aktivität von Leukozyten bestimmt werden, insbesondere von Granulozyten, Lymphozyten oder/und Monozyten.

Das erfindungsgemäße Verfahren ist insbesondere zur Bestimmung der Chemotaxis-Aktivität von neutrophilen Granulozyten geeignet. Aufgabe der neutrophilen Granulozyten ist die Beseitigung von eingedrungenen Bakterien und abgestorbenen körpereigenen Zellen durch Phagozytose, meist im Rahmen einer entzündlichen Reaktion.

Zelladhäsionsmoleküle sind Rezeptormoleküle, die auf Oberflächen von Zellen auftreten. Zumeist handelt es sich bei Zelladhäsionsmolekülen um Glykoproteine, die spezifisch an andere Zellen binden oder mit Bestandteilen der extrazellulären Matrix (ECM) in Wechselwirkung treten. Auf Zellen des Immunsystems finden sich insbesondere Zelladhäsionsmoleküle, die für die Adhäsion an Zielzellen verantwortlich sind. Das endotheliale Leukozytenadhäsionsmolekül 1 (ELAM-1) zeigt eine Strukturverwandtschaft mit gewissen Lectinen, dem epidermalen Wachstumsfaktor, und Komplement-Regulationsproteinen aus der Selektin- oder LECAM-Superfamilie und ist beispielsweise bei der Anheftung von Leukozyten an Entzündungsherde beteiligt.

Bevorzugt wird die Expression eines Zelladhäsionsmoleküls bestimmt, welches eine Lectindomäne umfasst, insbesondere eines Zelladhäsionsmoleküls ausgewählt aus L-Selektin, CD62, insbesondere CD62L oder CD62b, LECAM-1, Mel-14, LAM-1, Leu-8, TQ1, LEC.CAM-1, DREG56, GMP-140 und ELAM. In einer besonders bevorzugten Ausführungsform wird die Expression des Zelladhäsionsmoleküls LECAM-1 bestimmt. Die Änderung der Expressionsdichte des Zelladhäsionsmoleküls LECAM ist direkt mit der Aktivierung von neutrophilen Granulozyten nach Chemotaxinbindung korreliert. Die Expressionsänderung erfolgt quasi binär von einem Zustand hoher Expressionsdichte in einen Zustand niedriger Dichte nach Aktivierung. Intermediäre Zustände erzeugen daher zwei Gipfel im Histogramm oder CD62-Expression. Deshalb wird in einer bevorzugten Ausführungsform die mit der Steigerung der Chemotaxis-Aktivität einhergehende Abnahme der Expressionsdichte eines Zelladhäsionsmoleküls ausgewählt aus L-Selektin, CD62, insbesondere CD62L oder CD62b, LECAM-1, Mel-14, LAM-1, Leu-8, TQ1, LEC.CAM-1, DREG56, GMP-140 und ELAM, bevorzugt von und insbesondere LECAM, an der Zelloberfläche der Leukozyten, insbesondere Granulozyten, bestimmt.

In einer weiteren bevorzugten Ausführungsform wird die Expression und insbesondere die Änderung, bevorzugt eine Zunahme der Expressionsdichte des Adhäsionsmoleküls CD1 1 b bestimmt. Es wurde festgestellt, dass die Expressionsdichte des Adhäsionsmoleküls CD 11 b nach einer ChemotaxinBindung heraufreguliert wird. Dieser Effekt ist jedoch nicht so stark ausgeprägt wie die Herabregulierung von CD62L, sodass die Bestimmung von CD62L mehr bevorzugt ist.

Zur Bestimmung der Expression wird bevorzugt ein für das Zelladhäsionsmolekül spezifisches Bindemolekül, insbesondere ein spezifischer Antikörper, verwendet. Die Expression kann dann durch geeignete Markierungsgruppen, wie etwa Fluoreszenzmarkierungen, Latexbeads, Enzymmarkierungen oder ähnliches, sichtbar gemacht werden. Es können sowohl direkte als auch indirekte Markierungen verwendet werden.

In einer bevorzugten Ausführungsform wird die Probe mit einem Chemotaxis-Stimulans vorinkubiert. Bevorzugt wird als Chemotaxis-Stimulanz ein chemotaktisches Peptid zur Stimulierung von Leukozyten, insbesondere von Granulozyten, verwendet. Geeignete chemotaktische Peptide sind beispielsweise Peptide, die einen N-terminalen N-Formyl-Rest aufweisen, insbesondere Peptide ausgewählt aus der Gruppe bestehend aus fMLP (N-Formyl-Met-Leu-Phe) oder Strukturanaloga davon, z.B. N-Formyl-Met-Leu-Phe-Benzylamid, N-Formyl-Met-Leu-Phe-Methylester, N-Formyl-Met-Leu-Phe-Phe, N-Formyl-Met-Pheund N-Formyl-NorLeu-Leu-Phe. Besonders bevorzugt wird als Chemotaxis-Stimulans fMLP oder/und Interleukin-8 (IL-8) eingesetzt. Die Endkonzentration des Chemotaxis-Stimulans, insbesondere eines chemotaktischen Peptids, in der Probe beträgt bevorzugt 10⁻⁹ bis 10⁻⁵ Mol/l, bevorzugt 10⁻⁸ bis 10⁻⁶ Mol/l, insbesondere etwa 5 x 10⁻⁸ Mol/l. Bevorzugt erfolgt die Inkubation mit dem Chemotaxis-Stimulans bei einer Temperatur von 20 bis 50 °C, insbesondere 30 bis 40 °C, besonders bevorzugt etwa 36 bis 38 °C für 2 bis 120 Minuten, vorzugsweise 5 bis 60 Minuten, insbesondere 10 bis 30 Minuten und besonders bevorzugt etwa 20 Minuten. Es ist auch möglich, das erfindungsgemäße Verfahren in mehreren parallelen Ansätzen mit verschiedenen Inkubationszeiten oder/und bei verschiedenen Inkubationstemperaturen durchzuführen, sodass eine Zeitkinetik gemessen werden kann, die weitere Aussagen über die Chemotaxis-Aktivität zulässt.

Die Auswertung der Bestimmung der Expression des Zelladhäsionsmoleküls kann mit geeigneten Verfahren, etwa einem Durchflusszytometer, einem Bildanalysesystem (z.B. auf Mikroskopbasis) oder in einem Spektralfluorimeter (auch für Mikrotiterplatten geeignet) durchgeführt werden. Vorzugsweise erfolgt die Bestimmung in einem Durchflusszytometer, was eine objektiv quantifizierbare und reproduzierbare Auswertung ermöglicht.

Ein weiterer Vorteil des erfindungsgemäßen Verfahrens besteht darin, dass die Chemotaxis-Aktivität sowohl in einer aufbereiteten als auch in einer nicht aufgereinigten Körperflüssigkeit, insbesondere in Vollblut, gemessen werden kann. Dadurch kann die Messgenauigkeit weiter erhöht werden, da Aufreinigungs- bzw. Aufbereitungsschritte nicht zwingend erforderlich sind. Zur Bestimmung der Chemotaxis-Aktivität wird als Probe bevorzugt Vollblut und insbesondere antikoagoliertes, z.B. heparinisiertes Vollblut verwendet.

Im Vollblut kann insbesondere die Herabregulierung (Downregulierung) von CD62L gut gemessen werden.

Es ist aber auch möglich, eine aufbereitete Körperflüssigkeit, bevorzugt ein Leukozyten-reiches Plasma, zu verwenden. Bei Verwendung von Plasma kann neben der Herabregulierung auch die Durchwanderung einer Membran gut gemessen werden.

Als Probe wird insbesondere eine Körperflüssigkeit von Säugern, bevorzugt eine humane Probe, eingesetzt.

Das erfindungsgemäße Verfahren zur Bestimmung der Chemotaxis-Aktivität kann vorteilhaft mit weiteren Verfahren zur Bestimmung von LeukozytenFunktionen kombiniert werden. Auf diese Weise können weitere analytische oder/und diagnostische Informationen erhalten werden.

Beispielsweise kann weiterhin eine Form- oder/und Größenbestimmung der Leukozyten, insbesondere der Granulozyten, auf herkömmliche Weise erfolgen. Die Form- oder/und Größenänderung der Leukozyten wird bevorzugt in einem Durchflusszytometer bestimmt.

In einer bevorzugten Ausführungsform des erfindungsgemäßen Verfahrens werden die Leukozyten, insbesondere Granulozyten, nach einer Inkubation mit einem Chemotaxis-Stimulans durch eine poröse Membran geleitet. Bevorzugt wird eine poröse Membran mit einem definierten Öffnungsdurchmesser verwendet.

Dadurch kann auch die Zahl von durch eine poröse Membran gewanderten Leukozyten, insbesondere Granulozyten, bestimmt werden. Die Zellzählung wird durch Zugabe von Latexbeads in einem Durchflusszytometer durchgeführt.

Es wurde überraschenderweise eine Membran gefunden, die in sehr kurzer Zeit den Durchtritt vor allem von Granulozyten erlaubt, was für die Testdauer und damit den klinischen Einsatz sehr vorteilhaft ist. Eine Beschichtung von Membranen mit Collagen steigert den Unterschied zwischen Kontrollansatz ohne Chemotaxis-Stimulans, z.B. fMLP und Testansatz mit Chemotaxis-Stimulans, z.B. fMLP. Auch durch Verwendung von mit Fibronectin, Gelatine oder anderen mit Collagen verwandten Substanzen beschichteten Membranen konnten gute Ergebnisse erzielt werden.

Im Stand der Technik beschriebene und in der Praxis angewandte Methoden zur Messung von Chemotaxis umfassen beispielsweise die Verwendung von Membranen aus Zellulose. Bei diesen Verfahren wird der Abstand der Wanderungsfront von der Startoberfläche nach entsprechender panoptischer Färbung der Zellen gemessen. Eine andere Alternative sind Agarose-beschichtete Glasobjektträger. Die Zellen werden dabei in ein Stanzloch gefüllt und fMLP in das andere. Die Neutrophilen wandern im Spalt zwischen Agar und Glas in Richtung ansteigender Chemotaxinkonzentration, wobei die Entfernung der Wanderungsfront nach Färbung mikroskopisch gemessen wird. Eine weitere Alternative zur Bestimmung der Chemotaxis sind zellgefüllte Glaskapillaren und Agarschalen analog der Objektträgermethode. Die Chemotaxis-Untersuchungen mit diesen Verfahren sind bisher Speziallaboratorien vorbehalten. Das erfindungsgemäße Verfahren, insbesondere die einfache und reproduzierbare Kombination von Insert und Durchflusszytometrie ermöglicht hingegen die Bestimmung der Chemotaxis auf einfache Weise.

Die Erfindung betrifft deshalb ein Verfahren zur Bestimmung der Chemotaxis-Aktivität von Leukozyten in einer Probe, welches dadurch gekennzeichnet ist, dass man die Zahl von durch eine poröse Membran gewanderter Leukozyten bestimmt. Die Verknüpfung der Verwendung von Membranen sowie der Zytometrie zur Bestimmung der Chemotaxis-Aktivität ist im Stand der Technik nicht vorbeschrieben.

Mit diesem Verfahren kann die Chemotaxis-Aktivität von Leukozyten, insbesondere von Granulozyten, Lymphozyten oder/und Monozyten, besonders bevorzugt von neutrophilen Granulozyten, bestimmt werden. Zur Messung der Durchwanderung werden bevorzugt dünne Membranscheiben eingesetzt. Geeignete Membranen weisen bevorzugt einen Porendurchmesser in der Größenordnung von 1,0 bis 10 µm, mehr bevorzugt von 2,0 bis 4 µm, auf. Geeignete Membranmaterialien sind beispielsweise Polycarbonat oder Polyethylenterephthalat.

In einer Ausführungsform dieses Verfahren kann eine so genannte Boydenkammer eingesetzt werden. Dabei wird eine Membranscheibe, beispielsweise eine Membranscheibe aus Polycarbonat (z.B. Track-Elch-Membran, Fa. Nukleopore, Bestell-Nr. 150444) eingesetzt, die in eine Blind Well Chamber, bestehend aus einer in der Mitte aufgebohrten Plastikschraube und einem aufgebohrten und mit Innengewinde versehenen Acrylblock eingespannt. Bei dieser Ausführungsform muss jedoch darauf geachtet werden, dass die untere Kammer luftblasenfrei gefüllt wird und es müssen die Druckverhältnisse zwischen den Kompartimenten beachtet werden. Bevorzugt werden daher als poröse Membran, die durchwandert werden soll, Zellkultureinsätze verwendet. Insbesondere die Handhabung in Bezug auf Mehrfachansätze und das Befüllen der Kammer ist bei Verwendung von Zellkultureinsätzen auf einfache Weise durchzuführen. Geeignete Zellkultureinsätze sind beispielsweise Tissue Culture Inserts der Fa. Nunc mit einem Durchmesser von 10 mm und einer Porengröße von 3,0 µm, wobei die Membranen aus Polycarbonat bestehen, sowie Zellkulturinserts der Fa. Falcon mit einem Durchmesser von 6,4 mm und einer Porengröße von 3,0 µm. Solche Zellkultureinsätze können in Loch-Zellkulturplatten, beispielsweise in 24-Loch-Zellkulturplatten eingesetzt werden.

Bei Verwendungen von porösen Membranen ist jedoch häufig der Unterschied in der Durchwanderung mit und ohne Chemotaxin bei starken Schwankungen gering. Eine deutliche Verbeserung des Verfahrens kann dadurch erzielt werden, dass man die poröse Membran, insbesondere Zellkultureinsätze, mit einer Beschichtung versieht. Geeignete Beschichtungen umfassen Tween, Polyanionen, Gelatine, Fibronectin und Milchpulver. Die besten Ergebnisse wurden mit Collagen-beschichteten Membranen erhalten, weshalb diese Ausführungsform am meisten bevorzugt ist. Die Spezies, von der das Collagen stammt, kann beliebig gewählt werden. Beispielsweise ist Collagen von Mensch, Maus oder Kalb geeignet.

Die zur Verwendung zur Bestimmung der Chemotaxis-Aktivität von Leukozyten in einer Probe besonders bevorzugten Collagen-beschichteten porösen Membranen, können beispielsweise dadurch hergestellt werden, dass man eine geeignete Membran, beispielsweise eine Membran mit einer Porengröße von 1 µm bis 10 µm (z.B. aus Polycarbonat oder Polyethylenterephthalat) für 5 Minuten bis 2 Stunden, insbesondere für 20 Minuten bis 40 Minuten mit gelöstem Collagen (beispielsweise 1 mg Collagen / ml 0,1 %-iger Essigsäure) inkubiert und nach Waschen trocknet. Collagen-beschichtete Zellkulturinserts sind hinsichtlich der Zeitdauer des Testansatzes, hinsichtlich der Handhabung und der Auswertemethode besonders vorteilhaft. Um Collagen-beschichtete poröse Membranen zu erhalten, können die oben genannten porösen Membranen beispielsweise mit humanem Collagen oder Kälbercollagen beschichtet werden. Mit Mauscollagen IV beschichtete Zellkulturinserts auf Polyethylenterephthalatbasis sind von der Fa. Falcon erhältlich.

Vorteilhafterweise werden Zellkulturinserts verwendet, die nach unten konisch zulaufen, was eine Benetzung an der Außenseite durch Berührung von Platte und Insert verhindert.

Besonders vorteilhaft wird die Durchwanderung durch eine poröse Membran, insbesondere durch eine mit Collagen-beschichtete poröse Membran und die Bestimmung der Änderung der Expressionsdichte eines Zelladhäsionsmoleküls sowie ggf. der Formwandel (z.B. gemessen im Vorwärtsstreulicht) zur Bestimmung der Chemotaxis-Aktivität von Leukozyten kombiniert. Eine Kombination mit Zytometrie und Expressionsdichteänderung liefert den Vorteil, dass die Adhäsion an Endothelzellen funktionell geprüft werden kann.

Bevorzugt werden parallel mit steigender Sensitivität die Formänderung, die Abnahme der Expressionsdichte eines Zelladhäsionsmoleküls, insbesondere LECAM, und die Zahl der durch die Membran gewanderten Leukozyten, insbesondere Granulozyten, gemessen.

Die Kombination der drei Messgrößen Herabregulierung eines Zelladhäsionsmoleküls, z.b. von CD62L, Formänderung von Leukozyten, insbesondere Granulozyten und Durchwanderung einer Membran ist ein weiterer Gegenstand der Erfindung. Durch diese Kombination erhält man weitere Informationen über die Chemotaxis-Aktivität. Hierzu ist zu bemerken, dass z.b. bei Calciumentzug (EDTA-Zugabe) zwar noch eine Herabregulierung stattfindet, jedoch ohne Formänderung der Leukozyten und ohne Durchwanderung einer Membran, sodass die drei Messgrößen nicht zwangsweise korreliert sind, sondern sich ergänzende Informationen liefern.

Unabhängig von der Durchführung des erfindungsgemäßen Verfahrens ist es vorteilhaft, zur Kontrolle immer eine Probe eines gesunden Probanden mitzuführen.

Ein weiterer Gegenstand der vorliegenden Erfindung ist ein Reagenzienkit zur Bestimmung der Chemotaxis-Aktivität von Leukozyten, insbesondere Granulozyten, welcher dadurch gekennzeichnet ist, dass er einen spezifischen Bindepartner zum Nachweis der Expression eines Zelladhäsionsmoleküls sowie ein Chemotaxis-Stimulans enthält. Bevorzugt liegen die beiden Komponenten physikalisch getrennt voneinander vor. Die Komponenten des Reagenzienkits können z.B. fertige Lösungen sein oder auch Lyophilisate darstellen, aus denen der Anwender die Lösungen erst herstellt. Der Reagenzienkit kann weiterhin übliche Puffer und Hilfsstoffe umfassen.

Bevorzugt ist der in dem Reagenzienkit enthaltene spezifische Bindepartner ein Antikörper gegen das Zelladhäsionsmolekül, insbesondere ein Antikörper gegen den Cluster CD62b oder CD1 1b. Der Reagenzienkit kann weiterhin eine direkte oder indirekte Markierung, insbesondere eine Fluoreszenzmarkierung, enthalten.

Ein weiterer Gegenstand der Erfindung ist ein Verfahren zur Diagnose eines Immundefekts oder/und einer Leukozytenfunktionsstörung, insbesondere einer Granulozytenfunktionsstörung, welches dadurch gekennzeichnet ist, dass man die Chemotaxis-Aktivität von Leukozyten in einer Probe aus dem Patienten durch das erfindungsgemäße Verfahren zur Bestimmung der Chemotaxis-Aktivität bestimmt und in einem weiteren Ansatz die Chemotaxis-Aktivität einer negativen Kontrollprobe bestimmt und das Vorhandensein oder die Abwesenheit eines Immundefekts oder/und einer Leukozytenfunktionsstörung durch Vergleich der erhaltenen Werte ermittelt. Leukozytenfunktionsstörungen manifestieren sich z.B. in rezidivierenden Hautabszessen oder Wundheilungsstörungen.

Zur Diagnose eines Immundefekts oder/und einer Leukozytenfunktionsstörung kann das erfindungsgemäße Verfahren zur Bestimmung der Chemotaxis-Aktivität sowie der erfindunsgemäße Reagenzienkit ebenfalls eingesetzt werden.

Die bisherigen klinischen Ergebnisse an Patienten zeigen, dass v.a. die Expressionsdichte der Adhäsionsmoleküle verändert ist, d.h. der Zustand der in vitro nach Stimulation erreicht wird, bereits in vivo vorhanden ist. Dadurch ist das Rollen und die Adhäsion an die Endothelzellen (innere Auskleidung der Blutgefäße) für die nachfolgende Auswanderung aus dem Blutgefäß (postkapilläre Venole) verschlechtert. Die eigentliche Durchwanderung durch die Membran ist meist nicht beeinträchtigt. Daher kommt der Expressionsdichtebestimmung von CD62L und CD11b und auch anderen Adhäsionsmolekülen eine enorme Bedeutung zu.

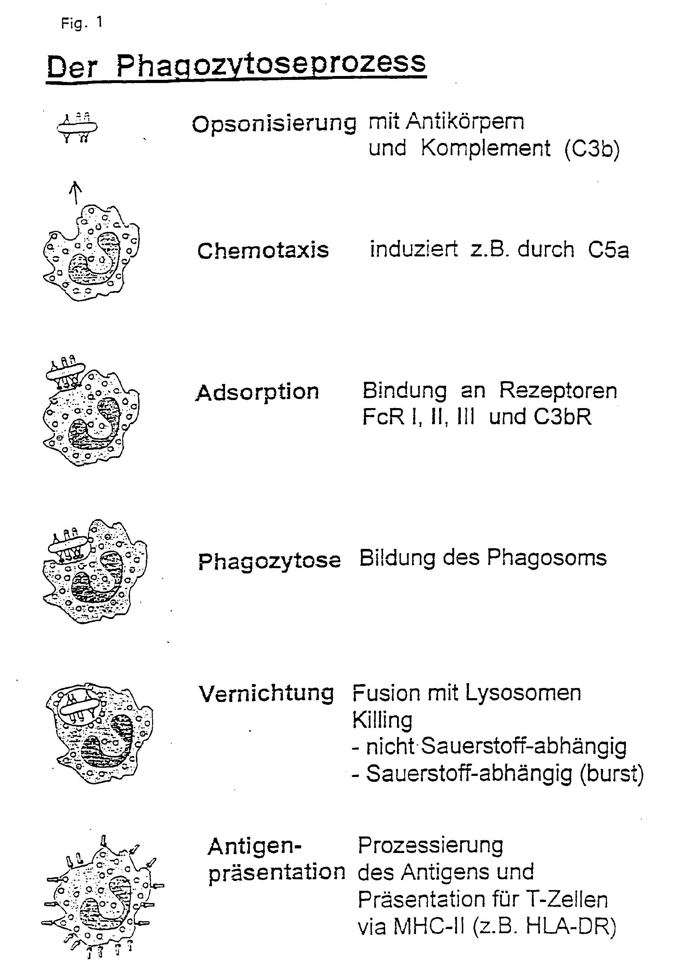

- Figur 1: zeigt die verschiedenen Schritte des Phagozytoseprozesses.

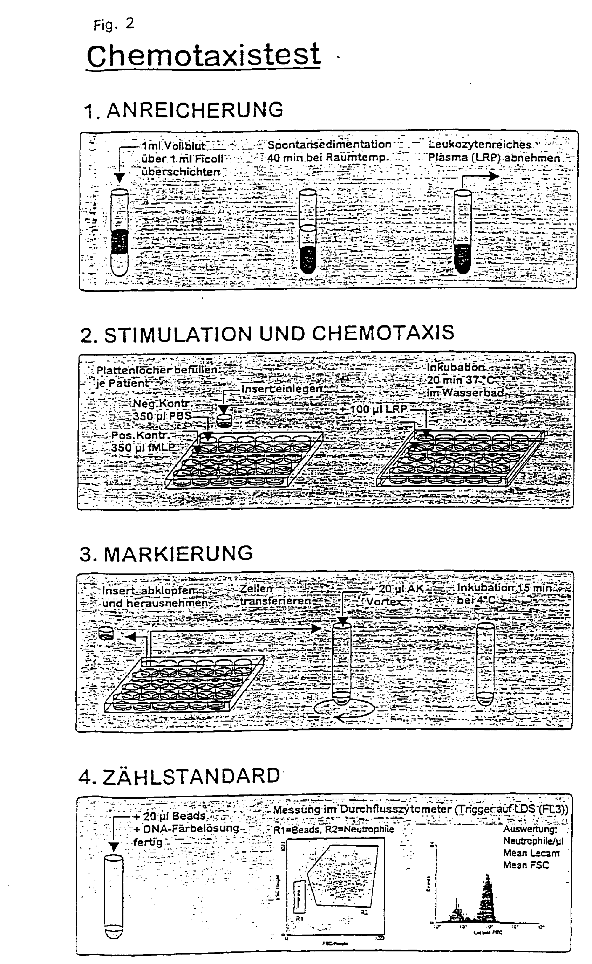

- Figur 2: zeigt eine schematische Darstellung des erfindungsgemäßen Chemotaxis-Tests.
- Figur 3: zeigt die mit einem Durchflusszytometer erhaltenen Ergebnisse.
- Figur 3A: zeigt die Negativkontrolle mit PBS, während Figur 3B einen Ansatz mit 5 x 10⁻⁸ MfMLP zeigt. Es ist eine Verringerung der Oberflächenexpression des Zelladhäsionsmoleküls LECAM-1 um etwa eine Größenordnung zu sehen. Die Zellzahl der neutrophilen Granulozyten (Region R2) wird in Relation zur konstanten Zahl von Beads (R1) gezeigt, wobei hier bei Stimulation (Fig. 3B) eine deutliche Erhöhung der durch die Membran gewanderten Zellen zu sehen ist.
- Figur 4: zeigt Grafiken zur Durchwanderung von verschieden beschichteten Membranen.
- Figur 4A: zeigt die Chemotaxis mit Polycarbonatmembranen der Fa. Nunc, die nicht, mit 0,1 % Milchpulver, mit 0,01 % Milchpulver, mit 1 % Tween 20 und mit 1 % Crodasinik O beschichtet wurden. Die Durchwanderung wurde jeweils in Abwesenheit (PBS) und in Anwesenheit (fMLP) eines Chemotaxis-Stimulans gemessen.
- Figur 4B: zeigt die Durchwanderung von Tween 20 bzw. Collagen-beschichteten Polyethylenterephthalat-Membranen.

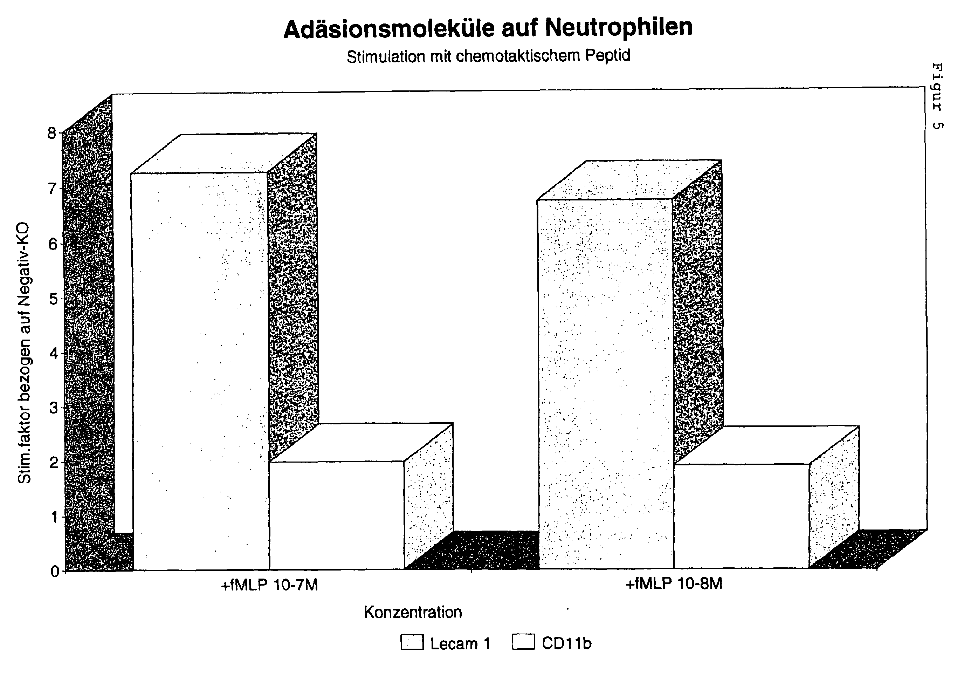

- Figur 5: zeigt Adhäsionsmoleküle auf Neutrophilen bei Stimulation mit chemotaktischem Peptid.

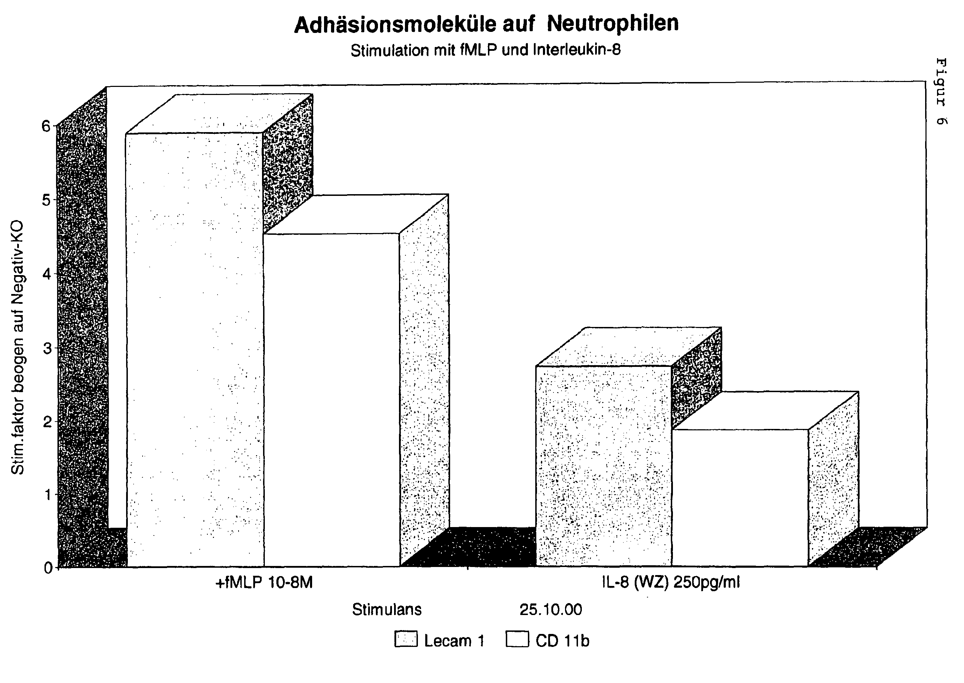

- Figur 6: zeigt Adhäsionsmoleküle auf Neutrophilen bei Stimulation mit fMPL und Interleukin-8.

Die Erfindung wird durch die folgenden Beispiele weiter erläutert.

### Beispiel 1

### Granulozyten-Funktionstest auf Chemotaxis aus Leukozyten-reichem Plasma

### 1.1 Anreicherung

1 ml Ficoll (Eicoll-Trennmedium, Firma Seromed, Bestell-Nr. L6113 (endotoxinarm)) wird in einem Eppendorf-Gefäß vorgelegt und 1 ml frisches (vorzugsweise nicht älter als 8 Stunden) Heparin-Vollblut (endotoxinarmes Heparin) wird vorsichtig bei Raumtemperatur überschichtet. Man lässt den Ansatz so lange bei Raumtemperatur stehen, bis eine deutliche Phasentrennung zu beobachten ist. Nach einer Spontansedimentation von üblicherweise etwa 40 Minuten bei Raumtemperatur verbleiben die Leukozyten und Thrombozyten über der Ficoll-Phase, während die Erythrozyten nach unten sedimentiert sind. Das Absinken der Erythrozyten tritt nach ca. 30 bis 40 Minuten recht plötzlich ein. Wird eine ältere Blutprobe, beispielsweise eine Blutprobe vom Vortag, verwendet, dauert der Sedimentationsvorgang wesentlich länger.

Ca. 500 µl der die obere Phase bildenden Zellsuspension werden abgenommen und gegebenenfalls auf Eis gelagert. Bei dieser Phase handelt es sich um ein Leukozyten-reiches Plasma (LRP).

### 1.2 Stimulation und Chemotaxis

In einer Zellkultur-Lochplatte (beispielsweise eine 24-Loch-Platte) werden pro Patient als Negativkontrolle 350µl PBS (phosphatgepufferte Salzlösung ohne Calcium und Magnesium; z.B. Firma Biochrom, Berlin) und für den Ansatz 350 µl 5 x 10⁻⁸ M fMLP (hergestellt aus einer 10⁻³ M Stammlösung, 1:20.000 vorverdünnt in PBS) vorgelegt. Zur Erhöhung der Genauigkeit sind mindestens zwei Ansätze pro Patient zu empfehlen. Daneben sollte zur Verbesserung der Testergebnisse neben dem Patienten ein gesunder Kontrollproband mitangesetzt werden.

Alternativ kann z.B. Interleukin 8 (IL-8) in einer Konzentration zwischen 1 und 100 U/ml als Stimulans eingesetzt werden.

In jedes Loch der Lochplatte wird eine Membran eingesetzt und auf diese je 100 µl der unter 1.1 erhaltenen Zellsuspension eingefüllt. Als Membran werden bevorzugt Zellkulturinserts mit definierter Porengröße verwendet. Die Zellsuspension kann beispielsweise mittels Pipette eingefüllt werden.

Zur Stimulation wird vorzugsweise 20 Minuten im Wasserbad bei 37 °C inkubiert. Dann werden die Zellen, die durch die Membran gewandert sind, geerntet. Dazu werden die Inserts abgeklopft, herausgenommen und die Zellsuspension in FACS-Röhrchen überführt. Die Röhrchen können zur Lagerung auf Eis gestellt werden.

### 1.3 Markierung

Zu den transferierten Zellen wird 10 µl des monoklonalen Antikörpers LECAM-1 FITC (z.B. Immunotech, Bestell-Nr. 1231) zugegeben und das Gemisch gevortext. Anschließend erfolgt eine Inkubation für 15 Minuten bei 4 °C auf Eis.

Alternativ kann 10 µl CD11b-FITC eingesetzt werden.

### 1.4 Messung

Der Lösung werden 20 µl LDS (Laserfarbstoff LDS 751, Firma Exiton oder Styryl 8, Firma Lambda Physik, Göttingen; zum Ausschluss der Erythrozyen, 1 mg/ml, vorverdünnt 1:50 in PBS) zugegeben. Parallel werden Zählpartikel (Calibrite Eichpartikel, Becton Dickinson, Bestell-Nr. 349502) mindestens 20 Sekunden gevortext und dem Ansatz 10 µl Zählpartikellösung zugegeben. Anschließend wird für ca. 10 Minuten inkubiert. Die Messung erfolgt danach an einem Durchflusszytometer (z.B. FACScan), wobei die Stabilität der Messprobe mindestens 2 Stunden auf Eis im Dunkeln beträgt. Die Messung wird nach Aufnahme von jeweils 1.000 bzw. 2.000 Latexpartikeln gestoppt, sodass ca. 5000 - 15000 Neutrophile gezählt wurden. Die Messung am Durchflusszytometer (z.B. FACScan) entspricht weitgehend der Vorgehensweise bei einer Immunfluoreszenzmessung zur Lymphozytentypisierung. Als Sheathflüssigkeit wird FACS-Flow (Firma Becton Dickinson, Bestell-Nr. 342003) oder eine andere Trägerflüssigkeit mit niedriger Eigenfluoreszenz verwendet. Die Verstärker- und Kompensationseinstellung erfolgt mit ungefärbten, FITC- und PE-gefärbten Mikropartikeln (Beads) über Autocomp-Software oder manuell. Es werden die PMT-Spannungen verändert. Die ungefärbten Beads liegen vom Nullkanal getrennt in der ersten Dekade und die FITC- bzw. PE-gefärbten Beads liegen jeweils auf ca. Mitte der Skala (Kanal 100). Die Eichung und Qualitätskontrolle für Fluoreszenzmessungen (Intensität pro Zelle) wird mit den Calibrites durchgeführt. Die Datenaufnahme und die Auswertung erfolgen mit dem CellQuest-Programm. Zur Auswertung wird ein Fenster um die Granulozytenpopulation gesetzt. Die Marker werden so gesetzt, dass die Auswerteregion den Bereich innerhalb der halbmaximalen Gipfelwerte einschließt. Weiterhin wird der Mittelwert der Intensität der Vorwärtslichtstreuung (FSC) der Granulozyten in der Darstellung Seitwärtsstreulicht (SCC) gegen Engwinkel-Vorwärtsstreulicht (FSC) betrachtet.

Eine zweite Region wird zur Zählung der Zellen um die Zellpartikel gesetzt und so lange aquiriert, bis hier 1.000 bzw. 2.000 Ereignisse genommen sind. Die Anzahl der Granulozyten in der PBS-Kontrolle und dem fMLP-Ansatz kann dann direkt miteinander verglichen werden.

Mit dem erfindungsgemäßen Verfahren wird die Fluoreszenzintensität bewertet, d.h. es wird eine quantitative Immunfluoreszenz gemessen.

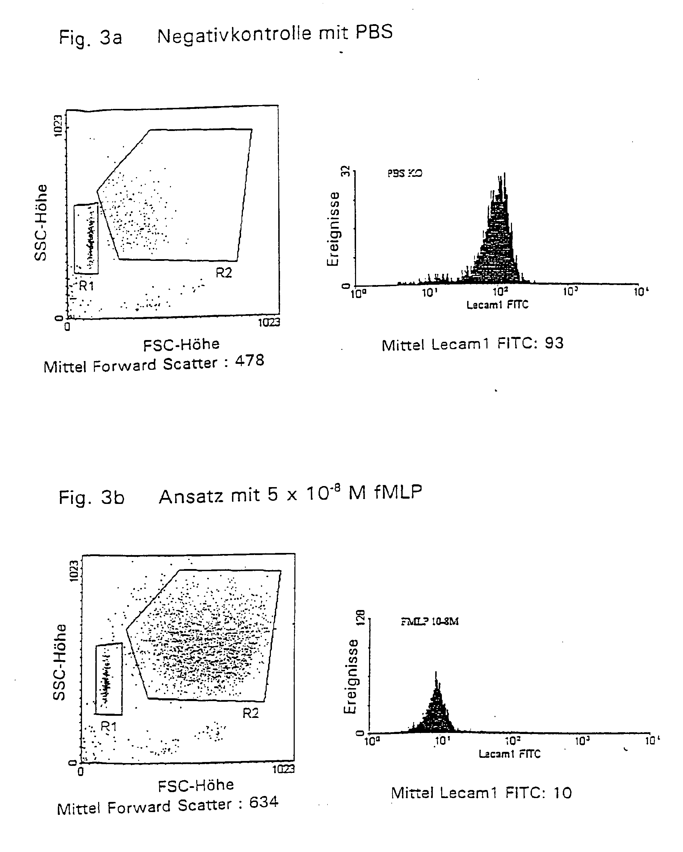

Beurteilt wird die Verringerung der LECAM-1-Fluoreszenz im fMLP- bzw. IL-8-Ansatz im Vergleich zum PBS-Ansatz beim Patienten und einem gesunden Kontrollprobanden sowie die Zunahme des FSC-Signals (vgl. Fig. 3).

Beim Einsatz von CD11b kommt es zu einer Zunahme der Expressionsdichte des Antigens. Die relative Änderung ist jedoch bei CD62 viel deutlicher ausgeprägt.

### Beispiel 2

### Chemotaxis-Test in Vollblut

Zur Durchführung der Bestimmung der Chemotaxis-Aktivität von Granulozyten in Vollblut wird das oben angegebene Protokoll folgendermaßen abgeändert:

Lithium-Heparinblut wird auf Eis gekühlt, um eine Voraktivierung der Granulozyten zu verhindern. In ein FACS-Röhrchen werden je Ansatz 100 µl Vollblut pipettiert. Zur Vollblutprobe werden dann 20 µl einer fMLP-Lösung (Vorverdünnung 1:5.000) oder 20 µl RPMI-Medium bzw. HBSS-Puffer (HBSS (Hanks gepufferte Kochsalzlösung; z.B. von Life Technologies, Katalog Nr. 24020)) als Negativkontrolle gegeben. Die Proben werden auf einem Vortexmischer kurz gemischt. Anschließend werden die Proben bei 37 °C 15 Minuten im Wasserbad inkubiert. Dann werden die Proben zum Abstoppen der Reaktion auf Eis gestellt. Es erfolgt die Zugabe von je 10 µl LECAM-1-FITC. Nach guter Durchmischung (Fortexen) werden die Proben im Eiswasserbad 15 Minuten unter Lichtausschluss inkubiert. Danach erfolgt die Zugabe von je 3 ml NH₄Cl-Lyselösung (NH₄Cl-Lyselösung (10 x Stammlösung in 1 l Aqua bidest 89,9 NH₄Cl, 10,0 g KHCO₃, 370,0 mg EDTA). Die Proben werden auf dem Vortexmischer gemischt und 5 Minuten bei Raumtemperatur inkubiert. Danach werden die Proben zweimal mit RPMI-Medium bzw. HBSS-Puffer durch Zentrifugation (5 Minuten, 250 x g) gewaschen. Danach erfolgt eine Zugabe von je 20 µl LDS und eine Inkubation von 5 Minuten auf Eis. Die Bestimmung der Abnahme der LECAM-1-Fluoreszenz und die Zunahme des FSC-Signals erfolgt mittels durchflusszytometrischer Messung.

### Beispiel 3

### Verwendung von beschichteten Membranen zur Messung der Durchwanderung

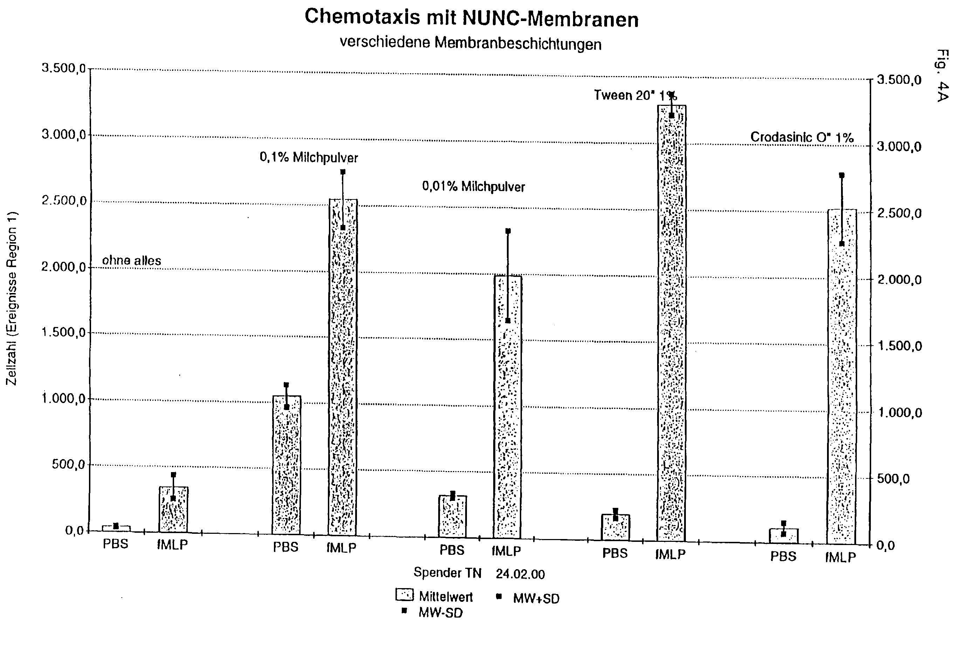

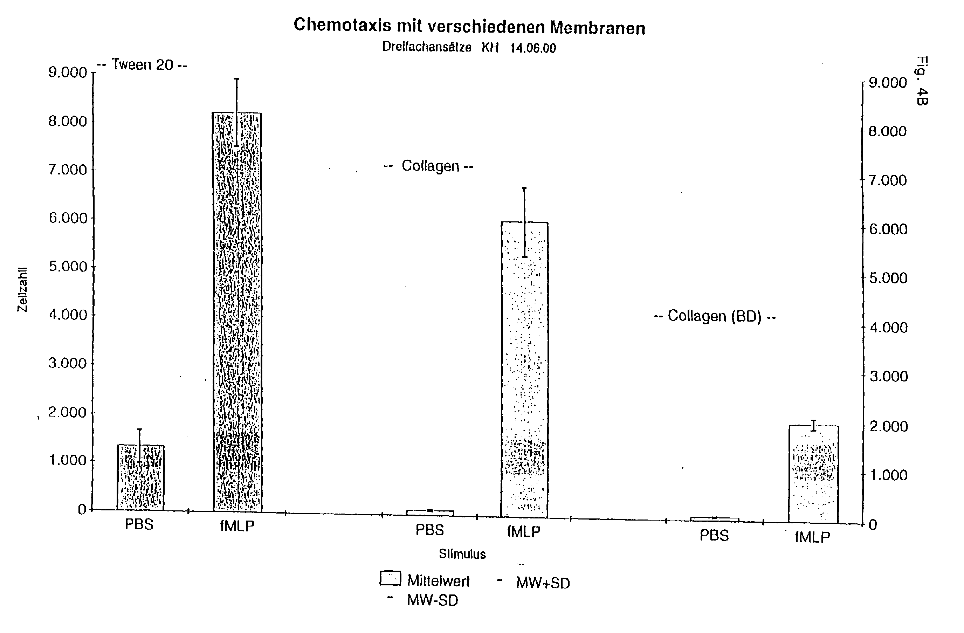

Die Chemotaxis-Aktivität wurde wie im Beispiel 1 beschrieben durchgeführt, wobei verschieden beschichtete Membranen in die Zellkultur-Lochplatte eingesetzt wurden. Die Beschichtungen der Membranen wurden durch Eintauchen der Membranen in verdünnte Lösungen der jeweiligen Beschichtungsmaterialien im PBS (phosphatgepufferte Salzlösung) für 30 Minuten aufgebracht und die Membranen anschließend im Wärmeschrank getrocknet. Fig. 4A zeigt Ergebnisse, die für verschiedene Membranbeschichtungen aufgebracht auf Polycarbonatmembranen (Tissue Culture Inserts der Fa. Nunc, Polycarbonatmembran, Bestell-Nr. 137370, Durchmesser 10 mm, Porengröße 3,0 µm) erhalten werden. Fig. 4B zeigt die Ergebnisse von der Verwendung eines kommerziell erhältlichen, mit Collagen beschichteten Zellkultureinsatzes (Collagen(BD); Zellkulturinserts der Fa. Falcon, PET (Polyethylenterephthalat) Membran, Bestell-Nr. 354545, Durchmesser 6,4 mm, Porengröße 3,0 µm, beschichtet mit Mauscollagen IV), sowie Polyethylenterephthalatmembrane der Fa. Falcon (Bestell-Nr. 353096), die mit Tween20 bzw. Collagen beschichtet wurden.

Wie aus Fig. 4A und Fig. 4B ersichtlich, kann mit beschichteten Membranen, insbesondere durch eine Beschichtung mit Collagen, eine deutliche Verbesserung der Ergebnisse, insbesondere eine deutliche Verbesserung der Unterschiede der Anzahl der die Membranen durchwandernden Zellen mit Stimulans (sMLP) und ohne Stimulans (nur PBS) erzielt werden.

## Patentansprüche

1. Verfahren zur Bestimmung der Chemotaxis-Aktivität von Leukozyten in einer Probe,
**dadurch gekennzeichnet,**
**dass** man die Zahl von durch eine poröse Membran gewanderter Leukozyten durch Zugabe von Latexbeads in einem Durchflusszytometer bestimmt.

2. Verfahren nach Anspruch 1,
**dadurch gekennzeichnet,**
**dass** man die Zahl von durch eine mit Collagen, Fibrinogen oder/und Gelatine beschichtete poröse Membran gewanderter Leukozyten bestimmt.

3. Verfahren nach einem der vorhergehenden Ansprüche,
**dadurch gekennzeichnet,**
**dass** man die Chemotaxis-Aktivität von Granulozyten, insbesondere neutrophilen Granulozyten bestimmt.

4. Verfahren nach einem der vorhergehenden Ansprüche,
**dadurch gekennzeichnet,**
**dass** man die Probe mit Chemotaxis-Stimulans insbesondere mit fMLP oder/und Interleukin-8 stimuliert.

5. Verfahren nach einem der vorhergehenden Ansprüche,
**dadurch gekennzeichnet,**
**dass** man eine Membran mit einem Porendurchmesser von 2,0 bis 4 µm einsetzt.

6. Verfahren nach einem der vorhergehenden Ansprüche,
**dadurch gekennzeichnet,**
**dass** man weiterhin die Expression eines Zelladhäsionsmoleküls bestimmt.

7. Verfahren nach Anspruch 6,
**dadurch gekennzeichnet,**
**dass** das Zelladhäsionsmolekül ausgewählt wird aus L-Selektin, CD62L, LECAM-1, Mel-14, LAM-1, Leu-8, TQ1, LEC.CAM-1, DREG56, GMP-140, ELAM und CD11b.

8. Verfahren nach Anspruch 6 oder 7,
**dadurch gekennzeichnet,**
**dass** die mit der Chemotaxis-Aktivität einhergehende Änderung der Expressionsdichte des Zelladhäsionsmoleküls an der Zelloberfläche der Leukoztyten bestimmt wird oder/und zur Bestimmung der Expression ein für das Zelladhäsionsmolekül spezifisches Bindemolekül verwendet wird.

9. Verfahren nach einem der vorhergehenden Ansprüche,
**dadurch gekennzeichnet,**
**dass** man zum Nachweis eine Fluoreszenzmarkierung einsetzt.

10. Verfahren nach einem der vorhergehenden Ansprüche,
**dadurch gekennzeichnet,**
**dass** als Probe antikoaguliertes Vollblut oder/und Leukozyten-reiches Plasma verwendet wird und/oder die Probe eine Humanprobe ist.

11. Verfahren nach einem der vorhergehenden Ansprüche,
**dadurch gekennzeichnet,**
**dass** weiterhin eine Form- oder/und Größenbestimmung der Leukozyten, insbesondere eine Bestimmung der Form- oder/und Größenänderung der Leukozyten in einen Durchflusszytometer erfolgt.

12. Reagenzienkit zur Bestimmung der Chemotaxis-Aktivität von Leukozyten, insbesondere von Granulozyten, in einem Durchflusszytometer,
**dadurch gekennzeichnet,**
**dass** er eine Collagen-beschichtete poröse Membran und weiterhin einen spezifischen Bindepartner zum Nachweis der Expression eines Zelladhäsionsmoleküls, insbesondere einen Antikörper gegen das Zelladhäsionsmolekül sowie ein Chemotaxis-Stimulans und gegebenenfalls eine direkte oder indirekte Fluoreszenzmarkierung und gegebenenfalls weiterhin übliche Puffer, Hilfs- oder/und Zusatzstoffe enthält.

13. Verfahren zur Diagnose eines Immundefekts oder/und einer Leukozytenfunktionsstörung,
**dadurch gekennzeichnet,**
**dass** man die Chemotaxis-Aktivität von Leukozyten in einer Probe aus dem Patienten durch ein Verfahren nach einem der Ansprüche 1 bis 11 bestimmt und in einem weiteren Ansatz die Chemotaxis-Aktivität einer negativen Kontrollprobe bestimmt und das Vorhandensein oder die Abwesenheit eines Immundefekts oder/und einer Leukozytenfunktionsstörung durch Vergleich der erhaltenen Werte ermittelt, wobei insbesondere ein Reagenzienkit nach Anspruch 12 verwendet wird.

## Claims

1. A method for determining the chemotactic activity of leukocytes in a sample, **characterized in that** the number of leukocytes which have migrated through a porous membrane is determined by adding latex beads in a flow-through cytometer.

2. The method as claimed in claim 1, **characterized in that** the number of leukocytes which have migrated through a porous membrane coated with collagen, fibrinogen and/or gelatin is determined.

3. The method as claimed in one of the preceding claims, **characterized in that** the chemotactic activity of granulocytes, in particular neutrophilic granulocytes, is determined.

4. The method as claimed in one of the preceding claims, **characterized in that** the sample is stimulated with a chemotaxis stimulant, in particular with fMLP and/or interleukin-8.

5. The method as claimed in one of the preceding claims, **characterized in that** a membrane having a pore diameter of from 2.0 to 4 µm is used.

6. The method as claimed in one of the preceding claims, **characterized in that** further the expression of a cell adhesion molecule is determined.

7. The method as claimed in claim 6, **characterized in that** the cell adhesion molecule is selected from L-selectin, CD62L, LECAM-1, Mel-14, LAM-1, Leu-8, TQ1, LEC.CAM-1, DREG56, GMP-140, ELAM and CD11b.

8. The method as claimed in claim 6 or 7, **characterized in that** the change in the expression density of the cell adhesion molecule, which change accompanies the chemotactic activity, is determined on the cell surface of the leukocytes or/and a binding molecule which is specific for the cell adhesion molecule is used for determining the expression.

9. The method as claimed in one of the preceding claims, **characterized in that** a fluorescence label is used for the detection.

10. The method as claimed in one of the preceding claims, **characterized in that** anticoagulated whole blood and/or leukocyte-rich plasma is used as a sample and/or the sample is a human sample.

11. The method as claimed in one of the preceding claims, **characterized in that** a determination of the shape or/and size of the leukocytes, in particular a determination of the change in shape or/and size of the leukocytes, is performed in a flow-through cytometer.

12. A reagent kit for determining the chemotactic activity of leukocytes, in particular of granulocytes, in a flow-through cytometer, **characterized in that** it contains a collagen-coated porous membrane and further a specific binding partner for detecting the expression of a cell adhesion molecule, in particular an antibody directed against the cell adhesion molecule, as well as a chemotaxis stimulant and optionally a direct or indirect fluorescence label and optionally further customary buffers, auxiliary substances or/and additives.

13. A method for diagnosing an immunological defect or/and a disturbance in leukocyte function, **characterized in that** the chemotactic activity of leukocytes in a sample from the patient is determined using a method as claimed in one of claims 1 to 11, and the chemotactic activity of a negative control sample is determined in another assay, and the presence or the absence of an immunological defect or/and a disturbance in leukocyte function is elucidated by comparing the obtained values, wherein in particular a reagent kit as claimed in claim 12 is used.

## Revendications

1. Procédé de détermination de l'activité de chimiotaxie de leucocytes dans un échantillon, **caractérisé en ce que** l'on détermine le nombre de leucocytes ayant migré à travers une membrane poreuse par ajout de perles de latex dans un cytomètre de flux.

2. Procédé selon la revendication 1, **caractérisé en ce que** l'on détermine le nombre de leucocytes ayant migré à travers une membrane poreuse enrobée de collagène, de fibrinogène et/ou de gélatine.

3. Procédé selon l'une des revendications précédentes, **caractérisé en ce que** l'on détermine l'activité de chimiotaxie de granulocytes, en particulier de granulocytes neutrophiles.

4. Procédé selon l'une des revendications précédentes, **caractérisé en ce que** l'on stimule l'échantillon avec un agent stimulant la chimiotaxie, en particulier avec de la fMLP et/ou de l'interleukine 8.

5. Procédé selon l'une des revendications précédentes, **caractérisé en ce que** l'on utilise une membrane présentant un diamètre de pores compris entre 2,0 et 4 µm.

6. Procédé selon l'une des revendications précédentes, **caractérisé en ce que** l'on détermine de plus l'expression d'une molécule d'adhésion cellulaire.

7. Procédé selon la revendication 6, **caractérisé en ce que** la molécule d'adhésion cellulaire est sélectionnée parmi la L-sélectine, CD62L, LECAM-1, Mel-14, LAM-1, Leu-8, TQ1, LEC.CAM-1, DREG56, GMP-140, ELAM et CD11b.

8. Procédé selon la revendication 6 ou 7, **caractérisé en ce que** l'on détermine la modification, provoquée par l'activité de chimiotaxie, de la densité d'expression de la molécule d'adhésion à la surface cellulaire des leucocytes et/ou **en ce que** l'on utilise pour déterminer l'expression une molécule de liaison spécifique de la molécule d'adhésion cellulaire.

9. Procédé selon l'une des revendications précédentes, **caractérisé en ce que** l'on utilise pour la détection un marquage par fluorescence.

10. Procédé selon l'une des revendications précédentes, **caractérisé en ce que** l'on utilise comme échantillon du sang entier anticoagulé et/ou du plasma riche en leucocytes, et/ou **en ce que** l'échantillon est un échantillon humain.

11. Procédé selon l'une des revendications précédentes, **caractérisé en ce que** l'on réalise en outre une détermination de la forme et/ou de la taille des leucocytes, en particulier une détermination de la modification de la forme et/ou de la taille des leucocytes dans un cytomètre de flux.

12. Kit de réactifs permettant de déterminer l'activité de chimiotaxie de leucocytes, en particulier de granulocytes, dans un cytomètre de flux, **caractérisé en ce qu'**il contient une membrane poreuse enrobée ou recouverte de collagène et en outre un partenaire de liaison spécifique permettant de détecter l'expression d'une molécule d'adhésion cellulaire, en particulier un anticorps dirigé contre la molécule d'adhésion cellulaire, ainsi qu'un agent stimulant la chimiotaxie et éventuellement un marquage par fluorescence direct ou indirect, et éventuellement en outre des tampons, excipients et/ou additifs usuels.

13. Procédé de diagnostic d'un déficit immunitaire et/ou d'une anomalie de la fonction leucocytaire, **caractérisé en ce que** l'on détermine l'activité de chimiotaxie de leucocytes dans un échantillon du patient par un procédé selon l'une des revendications 1 à 11, et que l'on détermine dans une seconde approche l'activité de chimiotaxie d'un échantillon de contrôle négatif, et **en ce que** l'on détermine la présence ou l'absence d'un déficit immunitaire et/ou d'une anomalie de la fonction leucocytaire par comparaison des valeurs obtenues, un kit ou ensemble de réactifs selon la revendication 12 étant en particulier utilisé pour ce faire.
